# EUROPEAN PATENT APPLICATION

(11) **EP 4 571 297 A1**
(43) Date of publication of application: **18.06.2025**
(21) Application number: 23847022.3
(22) Date of filing: 27.07.2023
(51) Int. Cl.: G01N 21/66, G01N 21/76, G01N 27/327, G01N 33/543

(54) **BIOSENSOR CARTRIDGE AND BIOSENSOR DEVICE COMPRISING SAME**

(30) Priority: 29.07.2022 KR 20220094517
(71) Applicant: Elips Diagnostics Inc., Seoul 06978 (KR); The General Hospital Corporation, Boston, MA 02114 (US)
(72) Inventor: SHIN, Ik-Soo, Seoul 03631 (KR)
(74) Representative: Dehns
(86) International application number: PCT/KR2023/010942
(87) International publication number: WO 2024/025365

(57) **Abstract**

A biosensor cartridge is disclosed. The biosensor cartridge according to an aspect of the present invention is a biosensor cartridge for detecting a biological material and generating an electrochemiluminescence signal, comprising: an electrode unit including a plurality of electrodes; and a compartment unit disposed on the electrode unit to compartmentalize an entire space provided on the electrode unit into a signal chamber and N reaction chambers surrounding the signal chamber; wherein the electrode unit comprises: a first electrode, at least a portion of which is disposed within the reaction chambers; a second electrode, at least a portion of which is disposed within the signal chamber; and N connecting electrodes disposed to electrically couple a chemical reaction within the reaction chamber to the electrochemiluminescence signal generated within the signal chamber.

## Description

### [Technical field]

The present invention relates to a biosensor cartridge, and more particularly, to a biosensor cartridge for detecting a biological material and generating an electrochemiluminescence signal, and a biosensor device including the same.

### [Background art]

Technically, *in vitro* diagnostic technologies are largely divided into molecular diagnosis technology that quantitatively and qualitatively analyzes specific genes, immunodiagnosis technology that analyzes specific antigens/proteins, clinical chemical analysis technology that analyzes the concentration of biological monomolecules, and extracellular vesicles (EV) analysis technology that analyzes EV occurs in the metabolic process of cells, etc. Such *in vitro* diagnostic technologies are widely used in various fields such as infectious diseases, diabetes, oncology, cardiology, autoimmune diseases, and new drug development.

On the other hand, regarding *in vitro* diagnostic technologies, there has been a paradigm shift from large hospital-centered diagnostic tests to patient-centered, distributed, and on-site diagnostic tests due to the increasing need for early diagnosis, preventive treatment, and health monitoring to diagnose diseases at an earlier stage.

In order to respond to this new paradigm, it is essential to develop an *in vitro* diagnostic device that can achieve the effects of high performance, decentralization, and miniaturization/on-site.

However, current *in vitro* diagnostic devices are polarized and supplied into two types: high-performance/large/expensive central laboratory diagnostic devices and low-performance/small/low-cost on-site diagnostic devices, and therefore do not have technical alternatives that can be applied to the new paradigm described above.

### [Disclosure]

### [Technical Problem]

The present invention is aimed at solving the above problems, and an object of the present invention is to provide a biosensor cartridge and a biosensor device comprising the same, which has a high sensitivity *in vitro* diagnostic performance and at the same time has a fast diagnostic speed and a compact structure so as to favor on-site supply.

The objects of the present invention are not limited to the tasks mentioned above, and other objects not mentioned will be clearly understood by those skilled in the art from the description below.

### [Technical Solution]

According to one aspect of the present invention, there is provided a biosensor cartridge for detecting a biological material and generating an electrochemiluminescence signal, comprising: an electrode unit including a plurality of electrodes; and a compartment unit disposed on the electrode unit to compartmentalize an entire space provided on the electrode unit into a signal chamber and N reaction chambers surrounding the signal chamber; wherein the electrode unit comprises: a first electrode, at least a portion of which is disposed within the reaction chambers; a second electrode, at least a portion of which is disposed within the signal chamber; and N connecting electrodes disposed to electrically couple a chemical reaction within the reaction chamber to the electrochemiluminescence signal generated within the signal chamber.

In this case, the connecting electrode may be integrally formed including a detection portion disposed in the reaction chamber and a luminescent portion disposed in the signal chamber.

In this case, the detection portion of the connecting electrode may be formed to wrap around at least a portion of the first electrode in a state spaced apart from the first electrode by a predetermined distance, and the luminescent portion of the connecting electrode may be disposed to face the second electrode, and may be formed to have a pointed end.

In this case, the connecting electrode may be formed to have a Y-shape as a whole.

In this case, the electrode unit may be formed in a circular shape, and the compartment unit may be formed such that its outer wall is circular to correspond to the shape of the electrode unit.

In this case, the compartment unit may comprise: an outer wall disposed at an outermost side of the compartment unit to form one side of the N reaction chambers; an inner wall disposed at an inner side of the outer wall to compartmentalize the signal chambers; and a partition wall extending from the inner peripheral surface of the outer wall to the outer peripheral surface of the inner wall, and spaced N apart along a peripheral direction of the inner wall.

In this case, the outer wall and the inner wall may be formed circularly, and eight partition walls may be equally spaced so that eight reaction chambers can be formed between the inner wall and the outer wall.

In this case, the second electrode may be formed to have an octagonal shape.

In this case, the inner wall may be disposed across the N connecting electrodes, and an insulator may be disposed in the portion of the N connecting electrodes where the inner wall is disposed.

In this case, the first electrode may include a support portion formed in the shape of a single closed curve and divided into N portions by the compartment unit.

In this case, the support portion may be formed in a circular shape centered on the second electrode.

In this case, the first electrode may further include N reaction portions protruding from the support portion in a direction toward the second electrode, and the N reaction portions may be disposed one by one in the N reaction chambers.

In this case, the reaction portion may be formed in a circular shape, and one end of the connecting electrode may be formed to surround the reaction portion in a state spaced apart from the reaction portion by a predetermined interval.

In this case, each of said N reaction chambers may be loaded with a different sample taken from a separate individual.

In this case, the same sample collected from the same individual may be introduced into the N reaction chambers, and different detection reagents may be introduced to detect a plurality of biological materials with respect to the same sample.

In this case, the biological material is a material for detecting the presence of an infectious disease, metabolic disease, immune disease, or cancer, and may be at least one of DNA, RNA, protein, metabolite, and extracellular vesicles, etc.

According to another aspect of the present invention, there is provided a biosensor device comprising: the aforementioned biosensor cartridge; a body unit in which a receiving space is formed to receive the biosensor cartridge; a voltage applicator disposed in the receiving space of the body unit and applying a voltage to the electrode portion of the biosensor cartridge; and an image sensor unit capturing the electrochemiluminescence signal generated in the second chamber of the biosensor cartridge.

In this case, the biosensor cartridge may be formed for disposable use.

In this case, the compartment unit may at least one insertion protrusion formed thereon projecting in an outward direction, and the body unit may have at least one insertion groove formed in a position corresponding to said insertion protrusion such that said insertion protrusion is inserted and secured therein.

In this case, the biosensor device may further comprise a magnetic application unit disposed in a position corresponding to said reaction chamber in the receiving space of the body unit to apply a magnetic force toward said reaction chamber.

### [Advantageous Effects]

According to the present constitutions described above, the biosensor cartridge according to an embodiment of the present invention can form optimized reaction conditions for each chamber through space separation through the compartment unit, so that highly sensitive detection results can be obtained.

In addition, the biosensor cartridge according to an embodiment of the present invention may be equipped with a plurality of reaction chambers so that a plurality of samples can be diagnosed simultaneously, and the speed of diagnosis can be dramatically improved by placing a plurality of luminescent portions in a single signal chamber so that the detection results can be confirmed in a single step.

In addition, the biosensor cartridge according to an embodiment of the present invention can further improve the sensitivity of detection by having a connecting electrode having an optimized structure for the detection portion and the luminescent portion.

In addition, the biosensor device according to an embodiment of the present invention can be operated with only miniaturized equipment, so it can be easily distributed to medical sites.

The effects of the present invention are not limited to the above effects, and should be understood to include all effects that can be inferred from the detailed description of the present invention or the constitutions of the invention described in the claims.

### [Description of Drawings]

FIG. 1 is an exploded perspective view illustrating a separated biosensor device according to an embodiment of the present invention.
FIG. 2 is a schematic configuration diagram of a biosensor device according to an embodiment of the present invention.
FIG. 3 is a view separately showing the front and rear sides of the biosensor cartridge according to an embodiment of the present invention.
FIG. 4 is a top view of the biosensor cartridge according to an embodiment of the present invention.
FIG. 5 is a view partially showing a portion of an electrode unit of the biosensor cartridge according to an embodiment of the present invention.
FIG. 6 is a diagram showing another example of an electrode unit of the biosensor cartridge according to an embodiment of the present invention.
FIG. 7 is a diagram illustrating connecting electrodes of the biosensor cartridge according to an embodiment of the present invention.
FIG. 8 is a diagram showing current density measured by varying the shape of a plurality of connecting electrodes included in the biosensor cartridge according to an embodiment of the present invention.
FIG. 9 is a diagram schematically illustrating a process of pre-processing a sample using magnet beads.
FIG. 10 is an explanatory diagram for explaining the spatial structure of a biosensor cartridge and chemical reactions in each chamber according to an embodiment of the present invention.
FIG. 11 is an explanatory diagram for explaining a spatial structure of a biosensor device to which a prior art is applied.
FIG. 12 is a diagram separately showing signal chambers before/after applying a voltage to the biosensor cartridge according to an embodiment of the present invention.

### [Mode of Invention]

Hereinafter, with reference to the accompanying figures, embodiments of the present invention will be described in detail so that those skilled in the art can easily carry out the present invention. This invention may be embodied in many different forms and is not limited to the embodiments set forth herein. In order to clearly describe the present invention, parts irrelevant to the description are omitted in the figures, and the same reference numerals are assigned to the same or similar constitutions throughout the specification.

The words and terms used in this specification and claims shall not be construed in their ordinary or dictionary sense, but shall be construed in a manner consistent with the technical idea of the invention, in accordance with the principle that the inventor may define terms and concepts in order to best describe his invention.

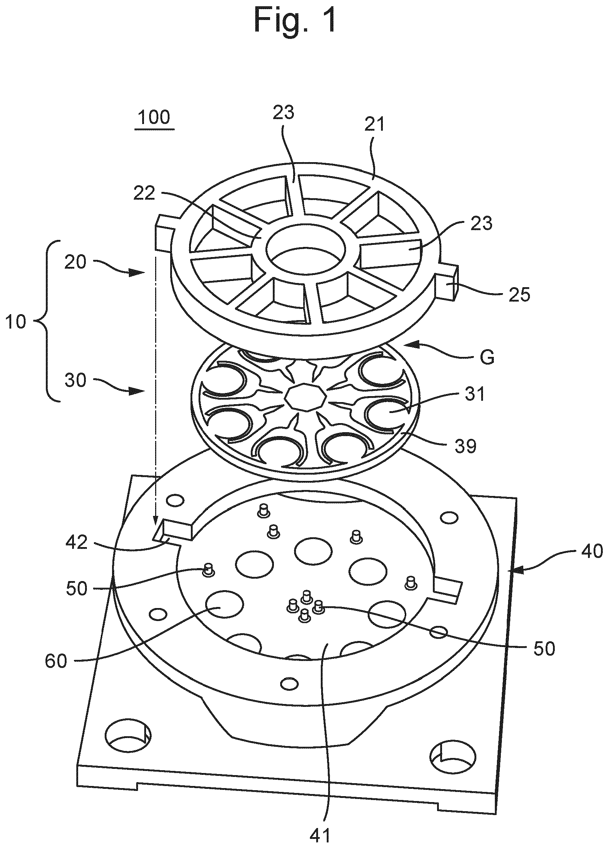

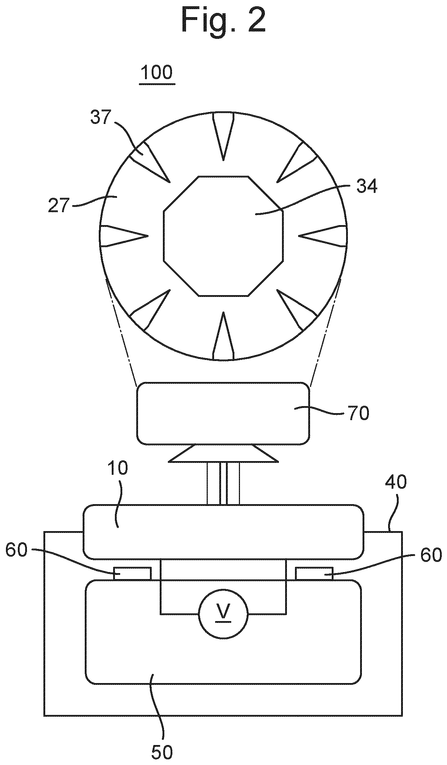

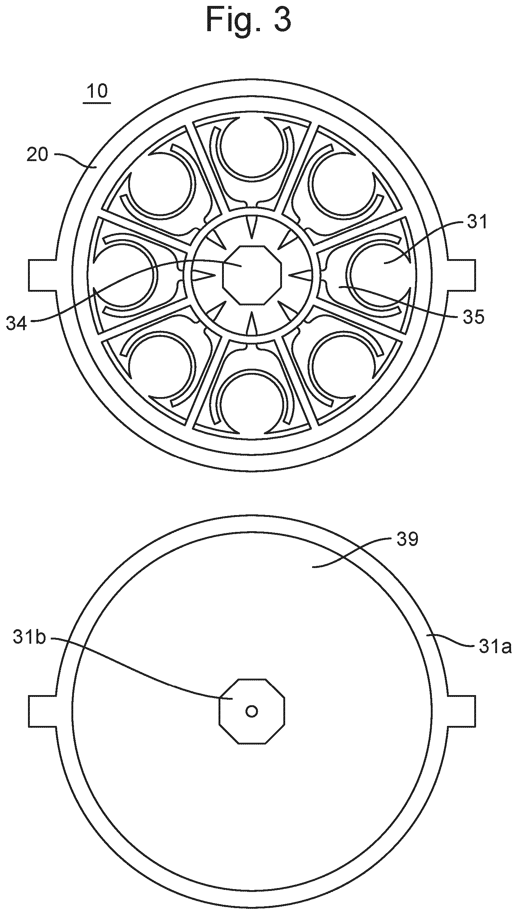

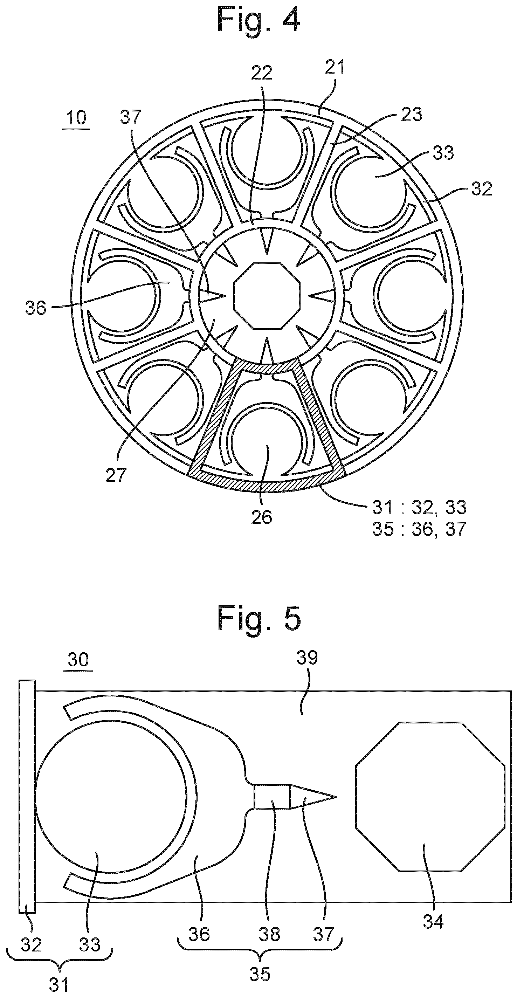

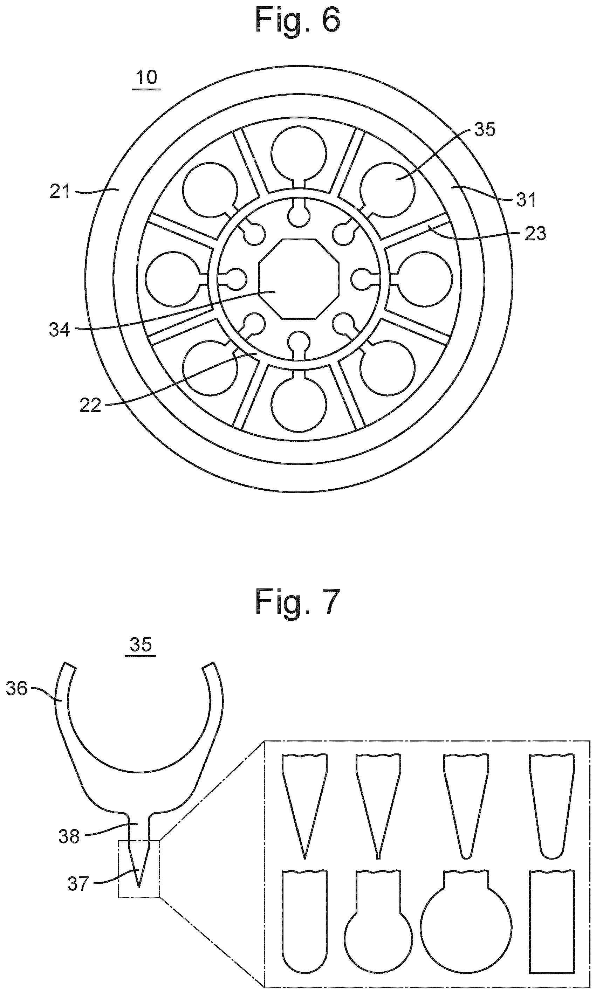

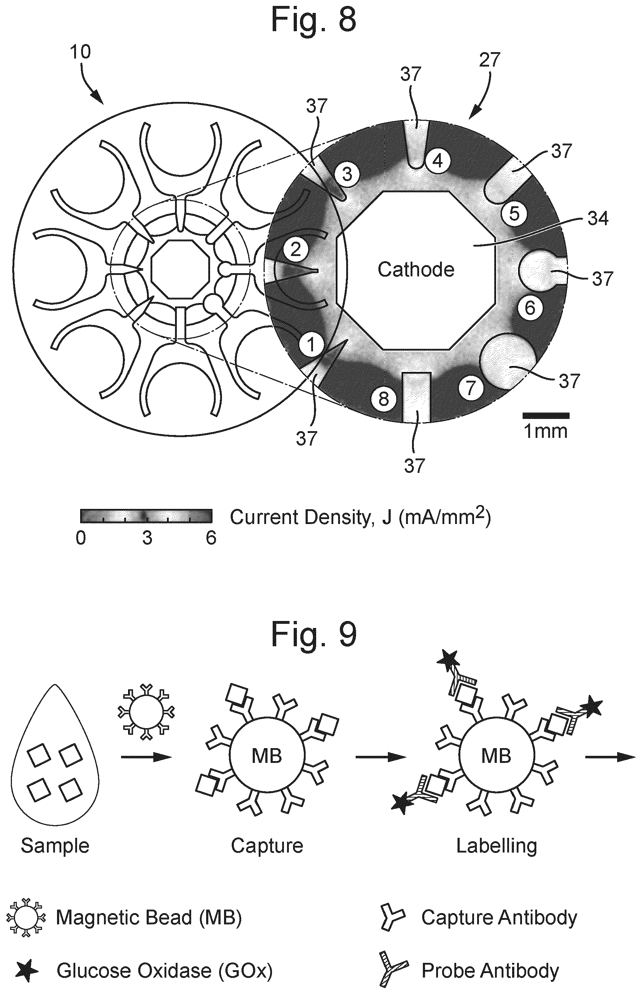

FIG.1 is an exploded perspective view illustrating a separated biosensor device according to an embodiment of the present invention. FIG.2 is a schematic configuration diagram of a biosensor device according to an embodiment of the present invention. FIG.3 is a view separately showing the front and rear sides of the biosensor cartridge according to an embodiment of the present invention. FIG.4 is a top view of the biosensor cartridge according to an embodiment of the present invention. FIG.5 is a view partially showing a portion of an electrode unit of the biosensor cartridge according to an embodiment of the present invention. FIG.6 is a diagram showing another example of an electrode unit of the biosensor cartridge according to an embodiment of the present invention. FIG. 7 is a diagram illustrating connecting electrodes of the biosensor cartridge according to an embodiment of the present invention. FIG. 8 is a diagram showing current density measured by varying the shape of a plurality of connecting electrodes included in the biosensor cartridge according to an embodiment of the present invention.

The biosensor device 100 according to one embodiment of the present invention is a device that can be utilized in various fields requiring the detection of biological material, such as in the field of medicine or research, and is a device that generates an electrochemical luminescence (ECL) signal in response to the detection of biological material contained in a sample.

In this case, the biosensor device 100 according to one embodiment of the present invention can improve the sensitivity of detection and at the same time rapidly diagnose the presence or absence of biomaterials in a plurality of samples by means of a biosensor cartridge 10 having a plurality of reaction chambers 26 and a singular signal chamber 27 spatially separated from each other. The following description will focus on the biosensor cartridge 10.

The biosensor cartridge 10 according to one embodiment of the present invention is a member in which a sample to be detected of a biomaterial is received, and may include a compartment unit 20 and an electrode unit 30, as shown in FIG. 1.

First, the compartment unit 20 is a member for compartmentalizing a total space G provided on top of the electrode unit 30 into a plurality of spaces 26,27, which will be described later.

More specifically, the compartment unit 20 may divide the entire space G into a signal chamber 27 disposed inside and a reaction chamber 26 disposed outside.

In this case, as shown in FIG. 4, the signal chamber 27 may be a space disposed in the center of the entire space G, and may exist in a singular number. In this case, a luminescent portion 37 of a connecting electrode 35 to be described later can be disposed in the signal chamber 27 so that an electrochemiluminescence signal can be observed. This will be described in detail through a corresponding part.

Next, unlike the signal chamber 27, a plurality of reaction chambers 26 may be provided. In this case, a plurality of reaction chambers 26 may be arranged to surround the central signal chamber 27. For example, 8 reaction chambers 26 may be provided as shown in the figure, but it should be noted that the number of reaction chambers 26 is not limited to the illustrated example. That is, if the number of reaction chambers 26 is two or more, various numbers (for example, 24) may be provided.

In addition, the figure shows that the electrode unit 30 is formed in a circular shape, and thus the entire space G is divided into a circular signal chamber 27 and a reaction chamber 26 in the shape of a truncated cone, but it is to be understood that the electrode unit 30 may have various other shapes, such as square, rectangular, and the like, and that the shapes of the signal chamber 27 and the reaction chamber 26 are not limited to the examples described above.

Meanwhile, a sample may be received in the reaction chamber 26, and when a voltage is applied to the electrode unit 30, a chemical reaction may be formed within the reaction chamber 26 depending on whether or not a biological material is included in the sample. For convenience of description, this will also be comprehensively described through the following sections.

In one embodiment of the present invention, the compartment unit 20 may be formed in a structure including an outer wall 21, an inner wall 22 and a partition wall 23 as shown in the figure.

First, the outer wall 21 is a portion disposed at the outermost side of the compartment unit 20 to form one side of the plurality of reaction chambers 26, which may be formed as a circle with a radius of size corresponding to the electrode unit 30, for example, as shown in the figure.

The inner wall 22 is a portion disposed inside the compartment unit 20 to compartmentalize the signal chamber 27, and may be formed in a circular shape having a smaller radius than the outer wall 21, for example.

Next, the partition wall 23 may be formed to extend from the inner peripheral surface of the outer wall 21 to the outer peripheral surface of the inner wall 22 so as to compartmentalize a space between the outer wall 21 and the inner wall 22. In this case, unlike the outer wall 21 and the inner wall 22, a plurality of partition walls 23 may be disposed, and preferably, they may be spaced apart at equal intervals to form signal chambers 27 of the same size.

As a specific example, as shown in the figure, when the outer wall 21 and the inner wall 22 are also formed in a circular shape corresponding to the circular electrode unit 30, eight reaction chambers 26 having the same volume can be formed with eight equally spaced partition walls 23. In this way, when both the outer wall 21 and the inner wall 22 are formed circularly, the plurality of reaction chambers 26 can all have the same volume and shape, which has the advantage that all reaction chambers 26 can be formed uniformly.

However, in the above description, the electrode unit 30 and the compartment unit 20 are both formed in a circular shape, but it is to be understood that the shape of the electrode unit 30 and the compartment unit 20 is not limited to this and may be formed in various shapes as described above.

In one embodiment of the present invention, the compartment unit 20 may be adhered to and fixed to the electrode unit 30 by means of, for example, an adhesive. This allows for an airtight seal between the compartment unit 20 and the electrode unit 30 to prevent fluid (sample or reagent) contained in the reaction chamber 26 or the signal chamber 27 from migrating to the other chamber through a small air gap formed at the bottom of the compartment unit 20.

In one embodiment of the present invention, the electrode unit 30 is a member disposed below the compartment unit 20 to form the signal chamber 27 and the reaction chamber 26 together with the compartment unit 20. In this case, the electrode unit 30 can include a plurality of conductive electrodes 31, 34, and 35, so that a voltage for inducing an electrochemiluminescence signal may be applied.

Specifically, the electrode unit 30 may include a plate 39 and a plurality of electrodes 31, 34, and 35.

First, the plate 39 may be formed of a known printed circuit board (PCB). In this case, the plate 39 may be formed in a plate shape having a predetermined thickness, and may be formed in a circular shape, for example, as shown in the figure.

In this case, the first electrode 31, the second electrode 34, and the connecting electrode 35 may be disposed on one surface of the plate 39 as shown in FIG. 3(a). In this case, the first electrode 31, the second electrode 34, and the connecting electrode 35 may be known electrodes formed of a conductive material to allow electricity to flow, and may be disposed in a patterned form on the plate 39, for example, by an electroless plating process.

In one embodiment of the present invention, the first electrode 31 is an electrode at least partially disposed within the reaction chamber 26, which may function as a medium for creating electrical potential energy in the reaction chamber 26 for a chemical reaction to occur in the reaction chamber 26.

As a specific example, the first electrode 31 may include a support portion 32 extending along an outer portion of the plate 39 as shown in FIG. 4, and may include a reactive portion 33 projecting from the support portion 32 in a direction toward the second electrode 34 to be described later.

In this case, the support portion 32 may be formed in a shape corresponding to the overall shape of the electrode unit 30, as shown in the figure, and may be formed to form a single closed curve shape such as a circle or a square.

As such, the single support portion 32 may be divided into a plurality of portions corresponding to the plurality of reaction chambers 26 by the compartment unit 20. In this case, each portion divided by the compartment unit 20 among the support parts 32 may be disposed in an individual reaction chamber 26.

When a voltage is applied to the support portion 32 by the voltage application unit 50, a chemical reaction including a sample can be made, and the plurality of reaction portions 33 form equal potential so that the plurality of reaction portions 33 can be electrically connected.

Next, the reaction portion 33 may be connected to a portion of the support portion 32 as a portion for expanding the area of the first electrode 31 to induce a more active chemical reaction when a voltage is applied to the first electrode 31, and may be formed in a circular shape as shown in FIG. 5, for example. In this case, the reaction portions 33 may be formed in a number corresponding to the number of reaction chambers 26 and disposed in each reaction chamber 26 one by one.

However, the first electrode 31 of the structure including the support portion 32 and the reaction portion 33 as shown in FIGS. 3 and 4 is only an example, and the first electrode 31 may also be formed with only the support portion 32 without the reaction portion 33 as shown in FIG. 6. In this case, the chemical reaction may sufficiently occur on the support portion 32.

In one embodiment of the present invention, a single number of second electrodes 34 may be disposed inside the signal chamber 27. In this case, voltage may be applied to the second electrode 34 by the voltage application unit 50 together with the first electrode 31. In this case, since a potential lower than that of the first electrode 31 is formed at the second electrode 34, a predetermined potential difference such as 0.4V may be formed between the first electrode 31 and the second electrode 34.

In this case, the second electrode 34 may be formed in a regular polygonal shape as shown in FIG. 5. For example, when the total number of reaction chambers 26 is compartmentalized by the compartment unit 20 so that there are eight reaction chambers, the second electrode 34 may be formed to have an octagonal shape to maintain uniformity without being electrically biased toward any one chamber.

Referring again to FIGs 3 through 5, between the first electrode 31 and the second electrode 34, a connecting electrode 35 may be disposed for electrically connecting the reaction chamber 26 and the signal chamber 27. To this end, the connecting electrode 35 may be formed as an overall integral structure with no physical separation as shown in the figures, but a portion 36 may be disposed in the reaction chamber 26 and another portion 37 may be disposed in the signal chamber 27. In other words, the connecting electrode 35 may be disposed to pass through both the reaction chamber 26 and the signal chamber 27, in which case the inner wall 22 of the compartment unit 20 may be disposed to pass across the connecting electrode 35.

Through these connecting electrodes 35, the reaction chamber 26 and the signal chamber 27 can be spatially separated by the compartment unit 20 to prevent fluid communication, yet electrically connected to each other. Here, being electrically connected may mean that the electrical operation of either of the signal chamber 27 and the reaction chamber 26 affects the electrical operation of the other.

In one embodiment of the present invention, the connecting electrode 35 may include a detection portion 36 disposed in the reaction chamber 26 together with the first electrode 31 and a luminescent portion 37 disposed in the signal chamber 27 together with the second electrode 34. In this regard, when a voltage is applied to the first electrode 31 and the second electrode 34, a chemical reaction may be formed in the detection portion 36 and, accordingly, an electrochemiluminescence reaction may be formed in the luminescent portion 37, which will be described later.

In this case, a plurality of connecting electrodes 35 may be disposed on the plate 39 to correspond to the number of reaction chambers 26. Further, the connecting electrodes 35 may be disposed so that their ends are spaced apart by a predetermined distance such that direct charge transfer is not possible with the first electrode 31 and the second electrode 34, respectively, as shown in the figure.

Referring to FIG. 4, when a plurality of connecting electrodes 35 are disposed on a plate 39, the detection portions 36 of the individual connecting electrodes 35 may each be disposed in any one of the plurality of reaction chambers 26. In contrast, the luminescent portion 37 may all be disposed in the signal chamber 27.

On the other hand, referring to FIG. 7 as an exemplary example related to the shape of the connecting electrode 35, the detection portion 36 of the connecting electrode 35 may be disposed at a predetermined distance from the reaction portion 33 of the first electrode 31, but may be formed to wrap around the reaction portion 33. By having the detection portion 36 of the connecting electrode 35 formed to wrap around the reaction portion 33 of the first electrode 31, the biosensor cartridge 10 according to one embodiment of the present invention can increase the electrode area over which a charge can move between the detection portion 36 and the reaction portion 33 of the first electrode 31, thereby inducing a more immediate movement of the charge on the connecting electrode 35 induced by the chemical reaction in the reaction portion 33 of the first electrode 31. As a result, the chemical reaction in the reaction chamber 26 and the electrochemiluminescence reaction in the signal chamber 27 can be synchronized more quickly and accurately.

Referring again to FIG. 7, the luminescent portion 37 of the connecting electrode 35 may be formed to have a variety of shapes. For example, it may be formed as a pointed tip as shown in the figure, or it may be formed as a circular shape similar to the reaction portion 33 of the first electrode 31, depending on the intensity or voltage of the intended electrochemical luminescence signal and the sensitivity of the image sensor unit 70 to be described later.

However, the inventors of the present invention have observed that when the electrode unit 30 and the compartment unit 20 were formed as shown in FIG. 4 (especially when the second electrode 34 had an octagonal shape), the most active electrochemiluminescence reaction was formed on the connecting electrode 35, which had a luminescent portion 37 with a pointed tip (see FIG. 8).

In summary, the connecting electrode 35 may be formed to have an overall Y-shaped shape, including a detection portion 36 of a structure bifurcated at both ends, and a luminescent portion 37 of a structure extending along one direction. As a result, the biosensor cartridge 10 according to one embodiment of the present invention can maximize the interconnection of the reaction chamber 26 and the signal chamber 27 via the detection portion 36, while also optimizing the electrochemiluminescence reaction in the luminescent portion 37.

In one embodiment of the present invention, referring to FIG. 4, an insulator 38 may be disposed to prevent electricity from passing through the portion of the connecting electrode 35 where the inner wall 22 of the compartment unit 20 is disposed (between the detection portion 36 and the luminescent portion 37). By disposing the insulator 38 on the lower part of the inner wall 22 in this manner, it is possible to block the direct electrical connection between the reaction chamber and the signal chamber 27, but to ensure that they are electrically connected only through the connecting electrode 35, thereby further improving the reliability of detection.

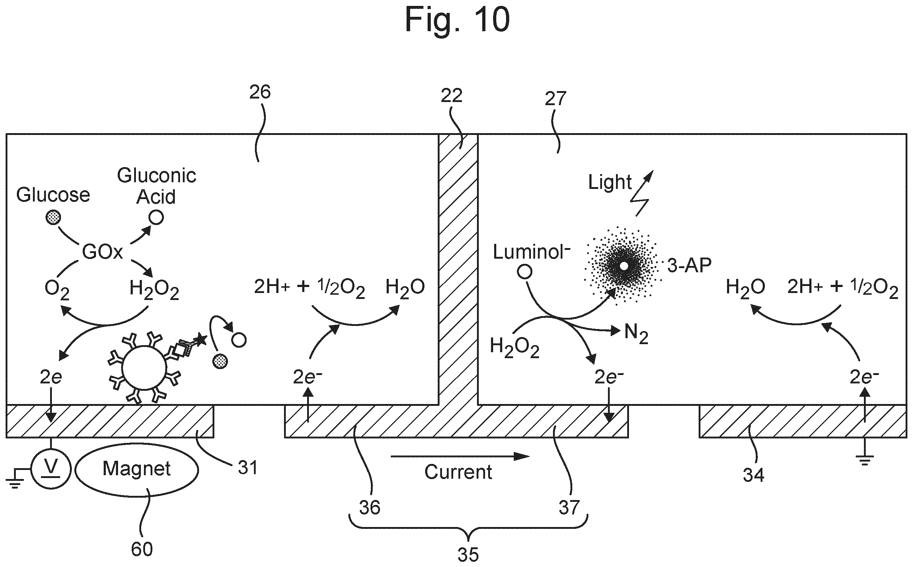

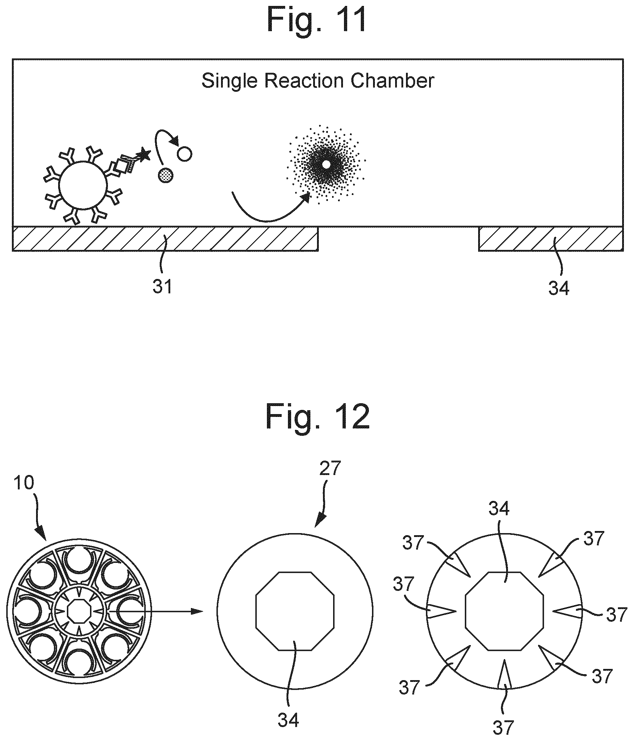

FIG. 9 is a schematic illustration of a sample pretreatment process using magnetic beads. FIG. 10 is an illustrative diagram to describe the spatial structure of a biosensor cartridge and the chemical reactions in each chamber according to one embodiment of the present invention. FIG. 11 is an illustrative diagram to describe the spatial structure of a biosensor device in the prior art. FIG. 12 is a separate illustration of the signal chamber before and after applying voltage to the biosensor cartridge according to one embodiment of the present invention.

Hereinafter, a chemical reaction and an electrochemiluminescence reaction in the reaction chamber 26 and the signal chamber 27 of the biosensor cartridge 10 according to an embodiment of the present invention will be described in detail with reference to the figures.

First, prior to introducing the sample into the reaction chamber 26 of the biosensor cartridge 10, a pretreatment process may be performed on the sample. That is, targets contained in the sample may be captured using immunomagnetic beads as shown in FIG. 9, and the captured targets may be further labeled with an oxidizing enzyme such as glucose oxidase (GOx) via probe antibodies.

The enzyme-labeled beads and glucose solution may then be added to the reaction chamber 26, and electrochemiluminescence reagents (ECL reagents) such as luminol and hydrogen peroxide (H₂O₂) may be added to the signal chamber 27.

Then, when a voltage is applied to the first electrode 31 and the second electrode 34 by the voltage application unit 50, the following chemical reaction may be formed in the reaction chamber 26. (See Fig. 10)

[Formula 1] - 1st electrode (anode): Glucose + O₂ → gluconic acid + H₂O₂ (GOx, Enzymatic reaction), H₂O₂ → O₂ + 2H+ + 2e-

- Detection portion of connecting electrode (cathode): 1/2 O₂ + 2H+ + 2e- → H₂O

In this case, in the reaction chamber 26, hydrogen peroxide is generated through the enzymatic reaction of glucose with the oxidase (GOx) of the enzyme-labeled bead, and the generated hydrogen peroxide is oxidized at the first electrode 31. Then, oxygen and hydrogen are reduced in the detection portion 36 of the connecting electrode 35. In this case, the number of electrons participating in the reaction becomes the same as the number of electrons participating in the reaction in the signal chamber 27.

The chemical reaction described above in the reaction chamber 26 may induce the following chemical reaction in the signal chamber 27.

[Formula 2] - Luminescent portion of connecting electrode (anode): Luminol + H₂O₂ → O₂ + 2H+ + N₂ + 2e- + 3-AP*, 3-AP* → 3-AP + *h* λ

- Second electrode (cathode): 1/2 O₂ + 2H+ + 2e- → H₂O

In this case, in the luminescent portion 37 of the signal chamber 27, an electrochemiluminescence reaction is induced in which chemical substances such as luminol or Ru(bpy) directly emit light based on the movement of electrons due to the chemical reaction induced in the reaction chamber 26. Therefore, depending on the type of reaction or degree of reaction occurring in the plurality of reaction chambers 26, the number of photons generated changes and the intensity of light changes.

In summary, when a voltage is applied to the first electrode 31 and the second electrode 34, the enzyme-labeled beads promote an oxidation-reduction reaction in the reaction chamber 26 to generate an electrochemiluminescence reaction in the signal chamber 27. In this case, the intensity of the electrochemiluminescence reaction in the signal chamber 27 is proportional to the concentration of the target protein. That is, as shown in FIG. 12, the luminescent portion 37, which was not luminescent before the voltage was applied to the electrode unit 30, forms an electrochemiluminescence reaction of different intensities depending on the concentration of the target protein contained in each reaction chamber 26 after the voltage is applied.

Thus, by spatially separating the reaction chamber 26, where the initial chemical reaction is formed, and the signal chamber 27, where the electrochemiluminescence reaction is formed, via the compartment unit 20, the biosensor cartridge 10 according to one embodiment of the present invention can perform each process under optimal reaction conditions for each chamber 26,27 without cross-torque, thereby achieving improved detection sensitivity.

More specifically, the chemical reactions in the reaction chamber 26 and the signal chamber 27 may have different optimal pHs, for example, GOx exhibits effective enzymatic activity at neutral pH, while electrochemiluminescence reagents (ECL reagents) such as luminol have increased solubility at basic pH, such as above pH 10. In view of this, the biosensor cartridge 10 according to one embodiment of the present invention can achieve a high signal-to-background noise ratio by forming optimal reaction conditions for both chambers 26 and 27 separately.

However, if the signal chamber and the reaction chamber are not spatially separated from each other as shown in FIG. 11, it is not possible to form a reaction condition that can optimize both reactions. In other words, in this case, it is only possible to form a reaction condition that is intermediate between the two optimal conditions described above, for example, pH 8.5. Therefore, a high signal-to-background noise ratio such as the present invention cannot be expected with a single-chamber structure.

It should be noted that the above-described pretreatment process or reagents in each chamber are only exemplary, and the application of the present invention is not limited to the above-described examples, i.e., designers may choose different optimized pretreatment processes and reagents depending on the target biological material.

The plurality of reaction chambers 26 of the biosensor cartridge 10 according to one embodiment of the present invention may be loaded with different types of samples. For example, if the biosensor cartridge 10 has eight reaction chambers 26, eight samples from eight different individuals can be introduced into each chamber. Although these different samples are introduced into each chamber, the luminescent portion 37, which indicates the detection of a target substance contained in the sample, is all disposed within a single signal chamber 27.

Thus, the biosensor cartridge 10 according to one embodiment of the present invention has the advantage of simultaneously testing a plurality of objects, while the results can be confirmed simultaneously by imaging only a single signal chamber 27, and the detection speed can be significantly improved with only a small device.

Hereinafter, a biosensor device 100 according to one embodiment of the present invention will be described.

Referring to FIGS. 1 and 2, a biosensor device 100 according to one embodiment of the present invention may include the biosensor cartridge 10 described above, a body unit 40, a voltage application unit 50, and an image sensor unit 70.

First, the body unit 40 is a member on which the biosensor cartridge 10 is mounted, as shown in FIG. 1, and may stably support the biosensor cartridge 10. For this purpose, the body unit 40 may have a receiving space 41 formed therein for insertion of the biosensor cartridge 10. Also, on one side of the receiving space 41 of the body unit 40, a correspondingly sized insertion groove 42 may be formed so that the insertion protrusion 25 protruding outwardly from the compartment unit 20 of the biosensor cartridge 10 can be inserted and fixed.

Next, in the receiving space 41 of the body unit 40, a voltage application unit 50 for applying a voltage to the electrode unit 30 of the biosensor cartridge 10 mounted in the receiving space 41 may be disposed. At this time, the voltage application part 50 may be electrically connected with the contact electrode 31a formed on the back side of the electrode part 30 through a terminal member such as an exposed electrode, a spring electrode, or a pogo pin.

On the other hand, at a position corresponding to the reaction chamber 26 of the electrode unit 30 in the receiving space 41, a magnetic application unit 60 may be provided for applying a magnetic force to the magnet bead in the reaction chamber 26. In this case, the magnetic application unit 60 may be disposed adjacent to the first electrode 31 to apply an attractive force to the magnetic beads to be positioned on the first electrode 31, thereby improving the density of the magnetic beads positioned on the first electrode 31 to induce a more active chemical reaction.

On the other hand, as shown in FIG. 2, an image sensor unit 70 may be disposed on the upper side of the biosensor cartridge 10 for photographing the electrochemiluminescence signal generated in the signal chamber 27. In this case, the image sensor unit 70 may be formed including a camera, an integrated circuit such as a charge-coupled device (CCD), a CMOS, or a disclosed image sensor such as a photoelectric amplifier tube (PMT).

On the other hand, the biosensor cartridge 10 described above may be formed as disposable to allow for immediate and repeated replacement when additional detection is required. This can further dramatically reduce the time required for detection.

As described above, the biosensor cartridge 10 according to one embodiment of the present invention is capable of obtaining a detection result of high sensitivity through spatial separation via the compartment unit 20. Furthermore, the biosensor cartridge 10 according to one embodiment of the present invention has a plurality of reaction chambers 26 so that a plurality of samples can be diagnosed simultaneously, but the speed of diagnosis can be dramatically improved by placing a plurality of luminescent portions 37 in a single signal chamber 27 so that the detection result can be confirmed in a single step. In addition, the biosensor device 100 according to one embodiment of the present invention has the advantage that it can be operated with equipment that is significantly miniaturized compared to conventional expensive equipment and can be easily distributed in the field.

Therefore, the biosensor device 100 according to one embodiment of the present invention can be effectively utilized for early diagnosis of severe diseases, including sepsis or COVID-19, that require rapid diagnosis, require expensive equipment, and require frequent diagnosis in the field.

While one embodiment of the present invention has been described above, the spirit of the present invention is not limited to the embodiment presented herein, and those skilled in the art who understand the spirit of the present invention will readily suggest other embodiments by adding, changing, deleting, adding constitutions, etc. within the scope of the same spirit, which will also be within the scope of the spirit of the present invention.

### [Explanation of symbols]

| | | | |
|---|---|---|---|
| 10: | Biosensor cartridge | 20: | Compartment unit |
| 30: | Electrode unit | 40: | Body unit |
| 50: | Voltage application unit | 60: | Magnetic application unit |
| 70: | Image sensor unit | 100: | Biosensor device |

## Claims

1. A biosensor cartridge for detecting a biological material and generating an electrochemiluminescence signal comprising:
an electrode unit including a plurality of electrodes; and
a compartment unit disposed on said electrode unit to compartmentalize an entire space provided on said electrode unit into a signal chamber and N reaction chambers surrounding said signal chamber;
wherein said electrode unit comprises:
a first electrode, at least a portion of which is disposed within said reaction chambers;
a second electrode, at least a portion of which is disposed within said signal chamber; and
N connecting electrodes disposed to electrically couple a chemical reaction within said reaction chamber to said electrochemiluminescence signal generated within said signal chamber.

2. The biosensor cartridge according to claim 1, wherein the connecting electrode is integrally formed including a detection portion disposed in the reaction chamber and a luminescent portion disposed in the signal chamber.

3. The biosensor cartridge according to claim 2, wherein the detection portion of the connecting electrode is formed to wrap around at least a portion of the first electrode in a state spaced apart from the first electrode by a predetermined distance, and the luminescent portion of the connecting electrode is disposed to face the second electrode, and is formed to have a pointed end.

4. The biosensor cartridge according to claim 3, wherein the connecting electrode is formed to have a Y-shape as a whole.

5. The biosensor cartridge according to claim 1, wherein the electrode unit is formed in a circular shape, and the compartment unit is formed such that its outer wall is circular to correspond to the shape of the electrode unit.

6. The biosensor cartridge according to claim 1, wherein the compartment comprises:
an outer wall disposed at an outermost side of the compartment unit to form one side of the N reaction chambers;
an inner wall disposed at an inner side of the outer wall to compartmentalize the signal chambers; and
a partition wall extending from the inner peripheral surface of the outer wall to the outer peripheral surface of the inner wall, and spaced N apart along a peripheral direction of the inner wall.

7. The biosensor cartridge according to claim 6, wherein the outer wall and the inner wall are formed circularly, and eight partition walls are equally spaced so that eight reaction chambers can be formed between the inner wall and the outer wall.

8. The biosensor cartridge according to claim 7, wherein the second electrode is formed to have an octagonal shape.

9. The biosensor cartridge according to claim 6, wherein the inner wall is disposed across the N connecting electrodes, and an insulator is disposed in the portion of the N connecting electrodes where the inner wall is disposed.

10. The biosensor cartridge according to claim 1, wherein the first electrode comprises a support portion formed in the shape of a single closed curve and divided into N portions by the compartment unit.

11. The biosensor cartridge according to claim 10, wherein the support portion may be formed in a circular shape centered on the second electrode.

12. The biosensor cartridge according to claim 10, wherein the first electrode further comprises N reaction portions protruding from the support portion in a direction toward the second electrode, and the N reaction portions are disposed one by one in the N reaction chambers.

13. The biosensor cartridge according to claim 12, wherein the reaction portion is formed in a circular shape, and one end of the connecting electrode is formed to surround the reaction portion in a state spaced apart from the reaction portion by a predetermined interval.

14. The biosensor cartridge according to claim 1, wherein each of said N reaction chambers is loaded with a different sample taken from a separate individual.

15. The biosensor cartridge according to claim 1, wherein the same sample collected from the same individual is introduced into the N reaction chambers, and different detection reagents are introduced to detect a plurality of biological materials with respect to the same sample.

16. The biosensor cartridge according to claim 1, wherein the biological material is a material for detecting the presence of an infectious disease, metabolic disease, immune disease, or cancer, and may be at least one of DNA, RNA, protein, metabolite, and extracellular vesicles.

17. A biosensor device comprising:
the biosensor cartridge according to any one of claims 1 to 16;
a body unit in which a receiving space is formed to receive the biosensor cartridge;
a voltage applicator disposed in the receiving space of the body unit and applying a voltage to the electrode portion of the biosensor cartridge; and
an image sensor unit capturing the electrochemiluminescence signal generated in the second chamber of the biosensor cartridge.

18. The biosensor device according to claim 17, wherein the biosensor cartridge is formed for disposable use.

19. The biosensor device according to claim 17, wherein the compartment unit comprises at least one insertion protrusion formed thereon projecting in an outward direction, and the body unit comprises at least one insertion groove formed in a position corresponding to said insertion protrusion such that said insertion protrusion is inserted and secured therein.

20. The biosensor device according to claim 17, further comprising a magnetic application unit disposed in a position corresponding to said reaction chamber in the receiving space of the body unit to apply a magnetic force toward said reaction chamber.
